# EUROPEAN PATENT APPLICATION

(11) **EP 2 641 533 A1**
(43) Date of publication of application: **25.09.2013**
(21) Application number: 11840820.2
(22) Date of filing: 15.11.2011
(51) Int. Cl.: A61B 5/00, A61B 5/1473, A61B 5/1486

(54) **SENSOR SYSTEM, AND METHOD FOR USING SENSOR SYSTEM**

(30) Priority: 16.11.2010 JP 2010256105
(71) Applicant: Terumo Kabushiki Kaisha, Tokyo 151-0072 (JP); Olympus Corporation, Shibuya-ku Tokyo 151-0072 (JP)
(72) Inventor: MATSUMOTO, Atsushi, Ashigarakami-gun Kanagawa 259-0151 (JP); HYUGA, Ryoji, Tokyo 151-0072 (JP)
(74) Representative: TBK
(86) International application number: PCT/JP2011/076306
(87) International publication number: WO 2012/067115

(57) **Abstract**

There is provided a sensor system and a sensor system using method which improve operability of a placing operation of placing a sensor in a living body and improve user-friendliness of users.

The sensor system 100 has: a sensor member 110 which has the sensor which detects analyte components of the living body and transmits a signal, and a retaining portion which retains the sensor; a needle assembly 210 which is detachably attached to the sensor member 110 and which has a guide needle which guides insertion of the sensor to the living body; a signal processing circuit member 310 which is detachably attached to the sensor member, and which has an electronic circuit which processes the signal from the sensor; and a fixing member which fixes the sensor member to a skin of the living body, and the sensor is placed in the living body in a state in which the signal processing circuit member is attached to the sensor member.

## Description

### TECHNICAL FIELD

The present invention relates to a sensor system which successively measures analyte components in a living body, and a method of using the sensor system.

### BACKGROUND ART

A sensor system is widely known which measures analyte components (body fluid component) by means of a sensor left in a living body. This sensor system inserts the sensor in a guide needle which punctures the living body, and embeds the sensor in the body upon puncturing by means of the guide needle. An electronic circuit such as a transmitter receives a signal transmitted according to the analyte components detected by the sensor to present to users information based on received content.

Patent Literature 1 discloses a sensor system which is provided with a sensor system main body and an electronic equipment unit which has an electronic circuit separately (see Patent Literature 1). The sensor system employs a configuration where the electronic equipment unit is attached to the sensor system main body after a puncturing operation of the guide needle, and a user attaches the electronic equipment unit while visually checking an attachment position.

### Citation List

### Patent Literature

Patent Literature 1: JP 2008-506468 W

### SUMMARY OF INVENTION

### Technical Problem

When a user places a sensor unit according to the conventional technique at a body surface portion which the user can hardly visually check, it is difficult to perform an attaching operation while checking the attachment position of an electronic equipment unit. The operation of attaching the electronic equipment unit becomes complicated, and there is a concern that failure to connect the electronic equipment unit and a sensor occurs.

There is a problem that the complicated operation of attaching the electronic equipment unit after a puncturing operation and a complicated operation of placing the sensor following the attaching operation cause a decrease in user-friendliness for the users who use a sensor system.

The present invention is made in light of the above problem, and an object of the present invention is to provide the sensor system and a sensor system using method which improve operability of the placing operation of placing the sensor in a living body and improve user-friendliness.

### Solution to Problem

A sensor system according to the present invention which achieves the above object has: a sensor member which has a sensor which detects an analyte component of a living body and transmits a signal, and a retaining portion which retains the sensor; a needle assembly which is detachably attached to the sensor member and which has a guide needle which guides insertion of the sensor to the living body; a signal processing circuit member which is detachably attached to the sensor member, and which has an electronic circuit which processes the signal from the sensor; and a fixing member which fixes the sensor member to a skin of the living body, and the sensor is placed in the living body in a state in which the signal processing circuit member is attached to the sensor member. Advantageous Effects of Invention

The sensor system according to the present invention configured as described above can perform a series of operations of placing a sensor in a living body in a state in which a signal processing circuit member which has an electronic circuit which processes a signal is attached to a sensor member which has the sensor and, consequently, does not require an operation of attaching the signal processing circuit member after a puncturing operation of a guide needle. Consequently, even when a user places the sensor at a body surface portion which the user can hardly visually check, it is possible to improve operability of an operation of placing the sensor without causing complication of the operation of attaching the signal processing circuit member, and thereby improve user-friendliness for the users who use the sensor system.

### BRIEF DESCRIPTION OF DRAWINGS

Fig. 1 is an outlook perspective view of a sensor system according to an embodiment.
Fig. 2 is an outlook perspective view of a signal processing circuit member, and Fig. 2(A) is a perspective view seen from a back surface side on which a sensor member is arranged to face with and Fig. 2 (B) is a perspective view seen from a top surface side.
Fig. 3 is an outlook perspective view of the sensor member, and Fig. 3 (A) is a perspective view seen from a back surface side on which the sensor member is arranged to face with a living body and Fig. 3(B) is a perspective view seen from a top surface side.
Fig. 4 is a view illustrating a simplified sensor member, and Fig. 4(A) is a plan view seen from a top surface side, Fig. 4(B) is an arrow view seen from an arrow 4B direction of 4(A) and Fig. 4(C) is a plan view seen from a back surface side.
Fig. 5 is a view for explaining a configuration of a sensor according to an embodiment, and Fig. 5(A) is a plan view illustrating an enlarged broken line portion 5A in Fig. 4(C) and Fig. 5(B) is a side view illustrating an example of a sensor probe.
Fig. 6 is an outlook perspective view of a needle assembly, and Fig. 6 (A) is a perspective view illustrating a state before the sensor member is attached and Fig. 6 (B) is a perspective view illustrating a state after the sensor member is attached.
Fig. 7 is a view for explaining the needle assembly, and Fig. 7(A) is an arrow view seen from an arrow 7A direction in Fig. 6 (B) and Fig. 7(B) is a view illustrating an enlarged broken line portion 7B in Fig. 6(A).
Fig. 8 is a view for explaining process of accommodating the sensor in an internal space of a guide needle, and Fig. 8(A) is a partially enlarged view of the back surface side of the sensor member illustrating a state before the sensor is accommodated in the guide needle, Fig. 8(B) is a partially enlarged view of the back surface side of the sensor member illustrating a state after the sensor is accommodated in the guide needle and Fig. 8(C) is a view for explaining a function of a slit of the guide needle and is an arrow view seen from an arrow 8C direction in Fig. 8(B).
Fig. 9 is a cross-sectional view along an arrow 9A-9A' line in Fig. 7(B).
Figs. 10(A) and 10(B) are cross-sectional views illustrating combinations of a cross-sectional shape of the guide needle and a cross-sectional shape of the sensor.
Figs. 11(A) to 11(C) are views for explaining an angle adjusting mechanism of a movable portion, and are views illustrating simplified arrow views seen from an arrow 11A direction in Fig. 1.
Figs. 12(A) and 12(B) are views for explaining process of separating the sensor member and the signal processing circuit member, and are views illustrating simplified cross sections along an arrow 12A-12A' line in Fig. 1.
Fig. 13 is a view illustrating a simplified sensor member and cover member, and Fig. 13(A) is a plan view seen from a top surface side, Fig. 13(B) is an arrow view seen from an arrow 14B direction in Fig. 13 (A) and Fig. 13 (C) is a plan view seen from a back surface side.
Figs. 14(A) to 14 (C) are views for explaining an adhesive portion and a peeling member, and are perspective views illustrating the connected needle assembly and the sensor member seen from a back surface side.
Fig. 15 is a view for explaining a function of the sensor system according to the embodiment, and Fig. 15(A) is a perspective view for explaining an operation of attaching the signal processing circuit member to the sensor member and Fig. 15(B) is a perspective view illustrating a state after the signal processing circuit member is attached.
Fig. 16 is a view for explaining the function of the sensor system according to the embodiment, and Fig. 16(A) is a perspective view illustrating a state before the peeling member which covers the adhesive portion provided in the movable portion is detached and Fig. 16 (B) is a perspective view illustrating a state after the peeling member which covers the adhesive portion provided in the movable portion is detached.
Fig. 17 is a view for explaining the function of the sensor system according to the embodiment, and Fig. 17(A) is a perspective view for explaining an operation of rotating the movable portion, Fig. 17(B) is a perspective view for explaining a puncturing operation and Fig. 17(C) is a perspective view for explaining an operation of releasing fixation of the needle assembly and the sensor member.
Fig. 18 is a view for explaining the function of the sensor system according to the embodiment, and Fig. 18(A) is a perspective view illustrating a state in which the fixation of the needle assembly and the sensor member is released and Fig. 18 (B) is a perspective view for explaining an operation of sliding the needle assembly and detaching the needle assembly from the sensor member.
Fig. 19 is a view for explaining the function of the sensor system according to the embodiment, and Fig. 19(A) is a perspective view illustrating a state after the needle assembly is detached from the sensor member and Fig. 19(B) is a perspective view illustrating a state in which the sensor member is fixed to a skin.
Fig. 20 is a cross-sectional view illustrating a sensor and a guide needle according to Modified Example.

### DESCRIPTION OF EMBODIMENTS

Hereinafter, an embodiment of the present invention will be described with reference to the drawings. In addition, dimension ratios in the drawings are exaggerated for ease of description, and are different from actual ratios in some cases.

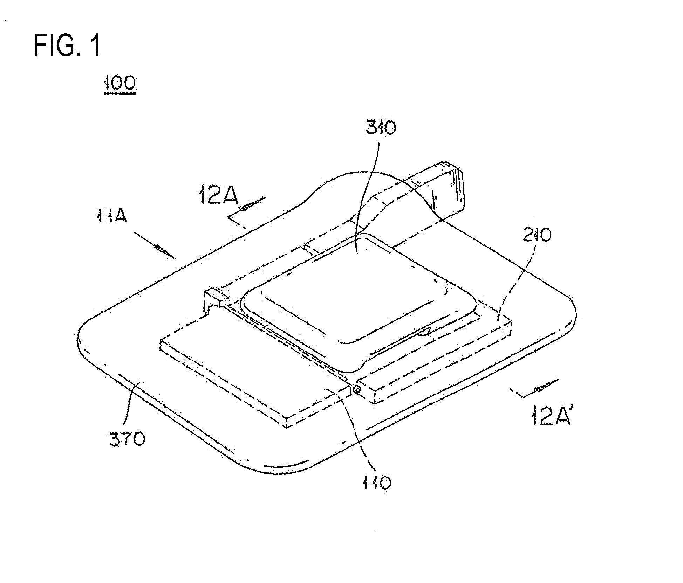

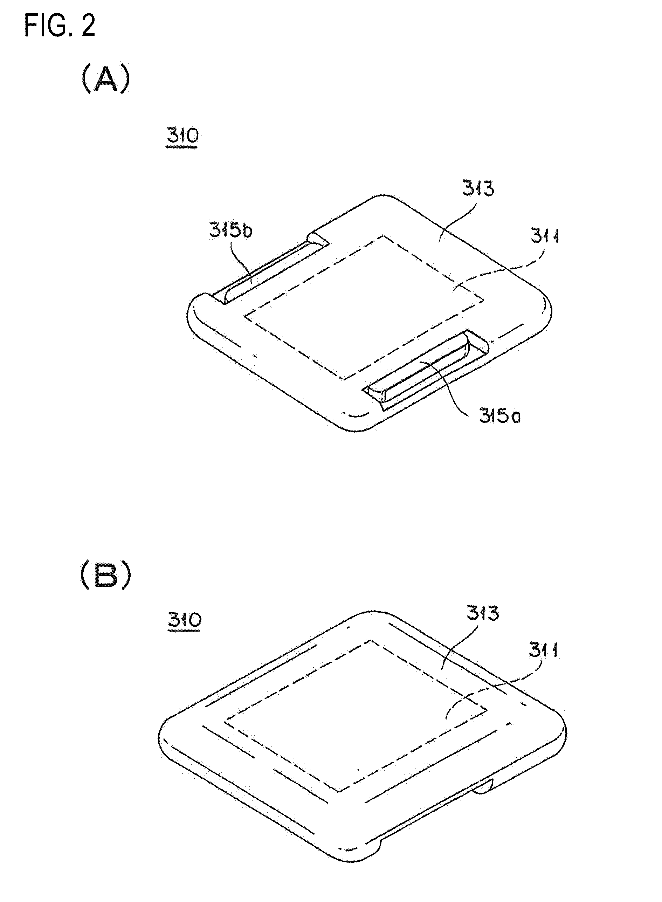

Referring to Figs. 1 and 2, a sensor system 100 is a sensor apparatus which can successively detect analyte components in a living body by means of a sensor inserted and embedded in the living body, measure and analyze the analyte components by means of an electronic circuit 311 of a signal processing circuit member 310 and present a measurement result to a user. The analyte components include, for example, glucose contained in an intercellular fluid, and the sensor system 100 can target at this glucose concentration as a measurement target and present the measurement result to a diabetic patient and the like who is the user. The measurement target analyte components include, for example, biological components such as lactic acid in addition to glucose. Further, the measurement targets are not limited to components in the living body alone and, for example, the sensor system 100 is applicable as a sensor which indicates physiological state such as a pH value.

A configuration of a sensor system according to the embodiment will be described.

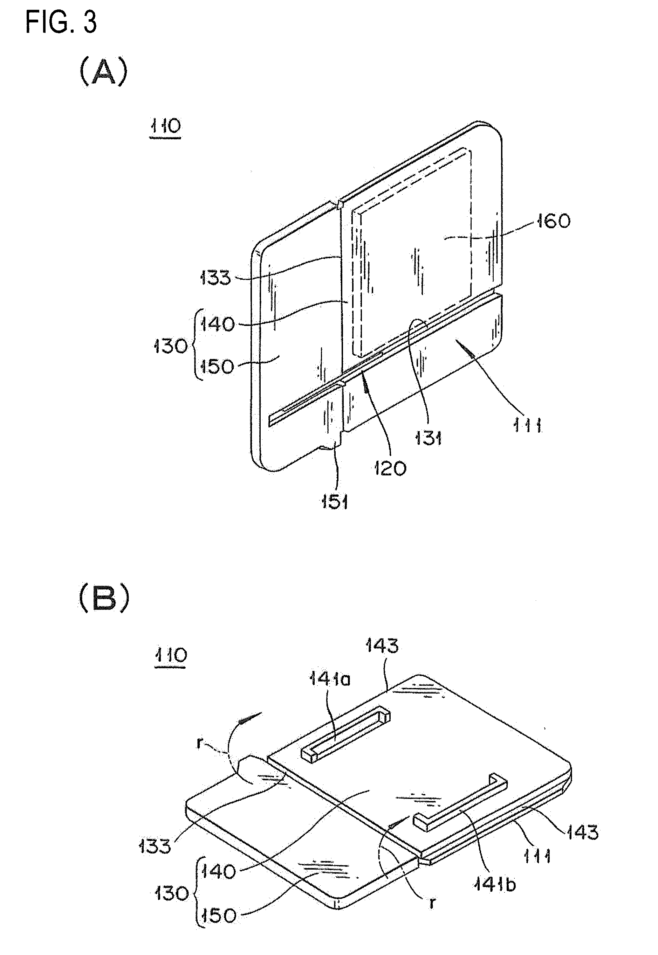

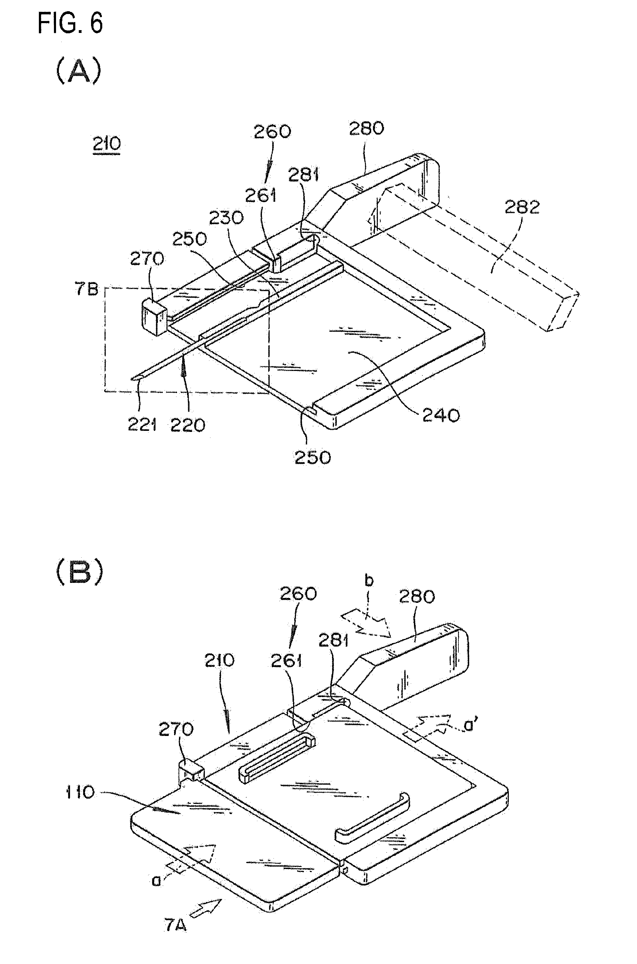

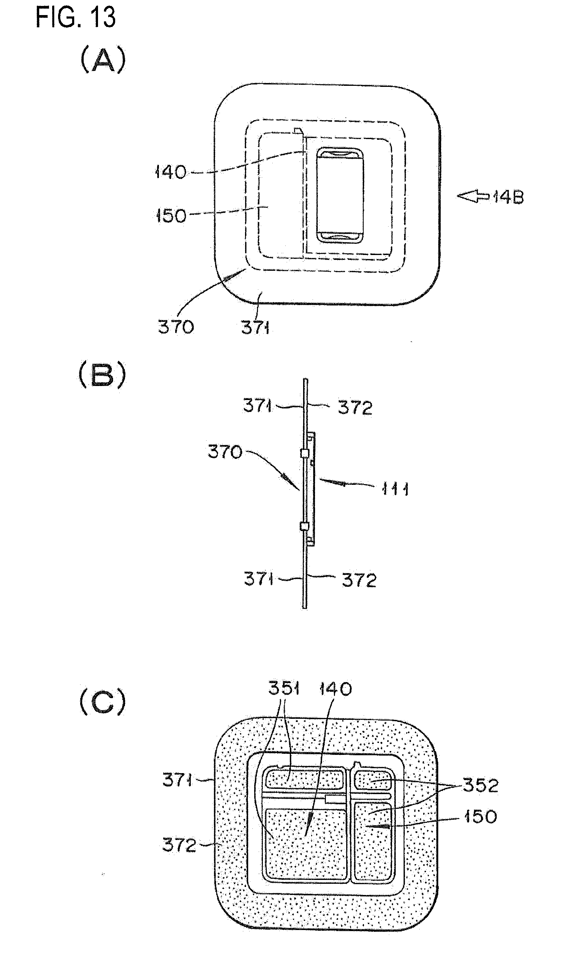

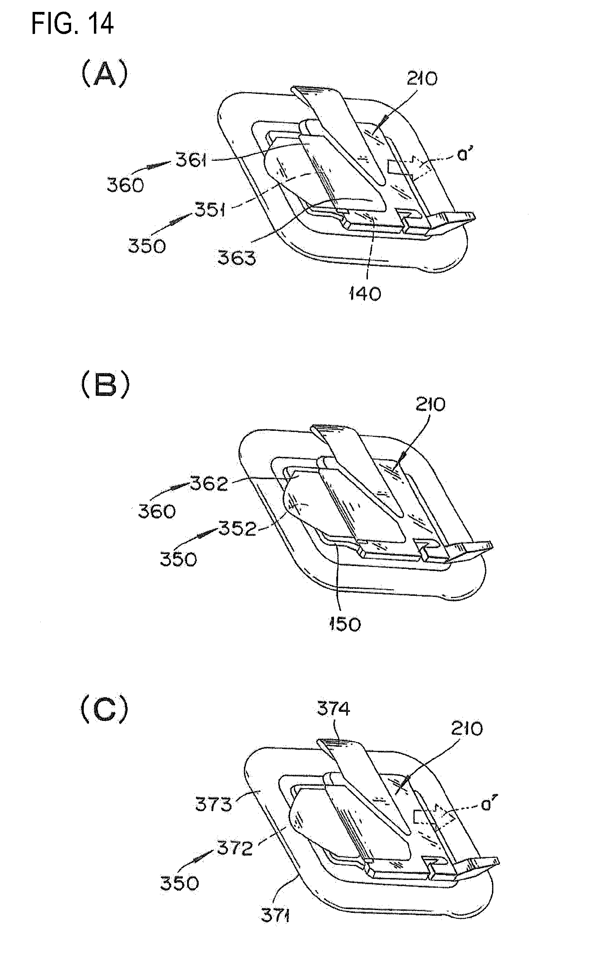

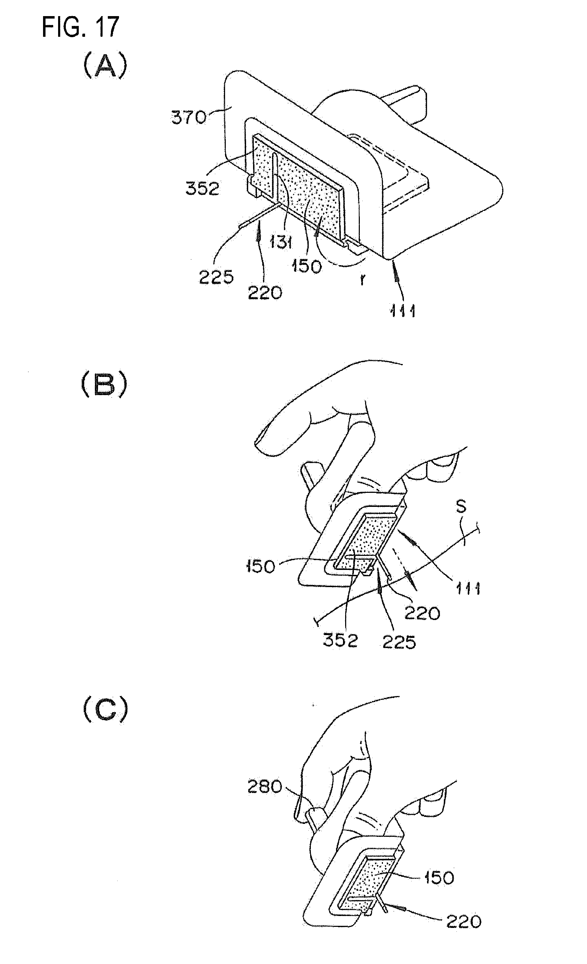

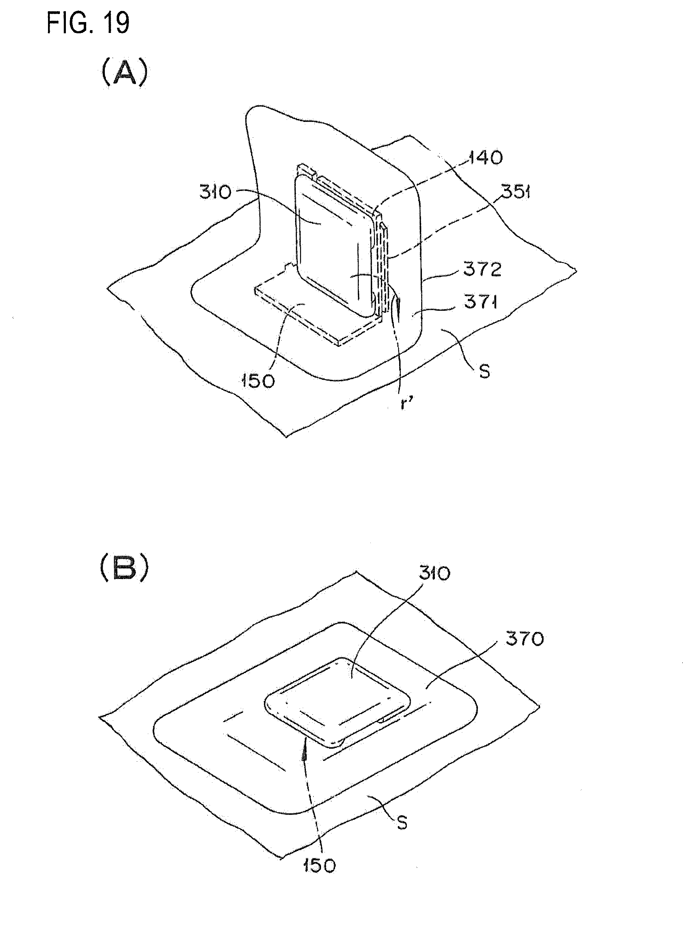

Referring to Fig. 1, the sensor system 100 according to the embodiment roughly has: a sensor member 110 which has a sensor 120 which detects analyte components of a living body and transmits a signal, and a retaining portion 130 which retains the sensor 120 (see also Fig. 3); a needle assembly 210 which is detachably attached to the sensor member 110 and which has a guide needle 220 which guides insertion of the sensor 120 to the living body (see also Figs. 6 and 17(B)); a signal processing circuit member 310 which is detachably attached to the sensor member 110, and which has an electronic circuit 311 which processes the signal from the sensor 120 (see also Fig. 2); and a fixing member 350 which fixes the sensor member 110 to a skin s of the living body (see also Figs. 13 and 14). Further, the sensor system 100 can place the sensor 120 in the living body in a state in which the signal processing circuit member 310 is attached to the sensor member 110 (see also Figs. 19(A) and 19(B)).

Referring to Figs. 2 (A) and 2(B), the signal processing circuit member 310 has a case 313 of a generally rectangular shape which accommodates the electronic circuit 311 therein. The case 313 can be made by utilizing, for example, hard plastic, metal or ceramic which can prevent a fluid from permeating or transmitting. For the hardplastic, for example, ABS resin, polypropylene, polycarbonate or polystyrene can be adopted. Further, for the metal, for example, titanium or SUS 316 can be adopted and, for the ceramic, for example, zirconia can be adopted. Although an outer shape dimension can be designed to various sizes according to functions to be added, for example, an outer shape dimension which is formed to have the width of 10 mm to 50 mm, the depth of 10 mm to 50 mm and the thickness of 1 mm to 10 mm can be used.

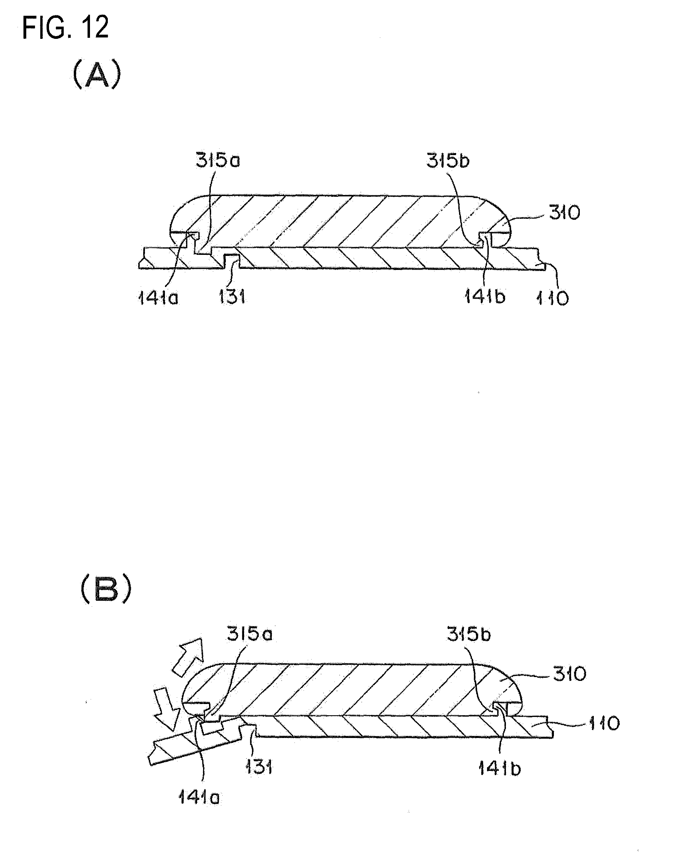

Fitting ribs 315a and 315b which connect with the sensor member 110 are provided on a back surface of the case 313. The back surface of the case 313 refers to a surface on which the sensor member 110 is arranged to face with (see Fig. 12).

The electronic circuit 311 of the signal processing circuit member 310 receives the signal transmitted from the sensor 120 provided in the sensor member 110, and executes predetermined processing based on content of this signal. The sensor system 100 takes in and processes the signal transmitted by the sensor 120 once in an electronic circuit 160 arranged in a main body portion 140 of the retaining portion 130 which forms a housing of the sensor member 110, and then transmits the processed signal to the electronic circuit 311 arranged in the signal processing circuit member 310 (see Fig. 3(A)).

A communication scheme between the electronic circuit 311 of the signal processing circuit member 310 side and the electronic circuit 160 of the sensor member 110 side can adopt a conventionally known technique. It is possible to adequately adopt, for example, a communication scheme of electromagnetic induction using dielectric coils, a communication scheme using radio waves or an optical communication scheme using LEDs and photodiodes placed to face with the LEDs. By adopting these communication schemes, it is possible to suitably prevent breakdown or occurrence of communication failure due to leakage of electricity even when the user takes a bath, takes a shower or swims. When a countermeasure of leakage of electricity is sufficiently taken, it is possible to adopt an electrical connecting scheme. By adopting such a connecting scheme, it is possible to transmit and receive signals according to a general communication scheme using electrical signals.

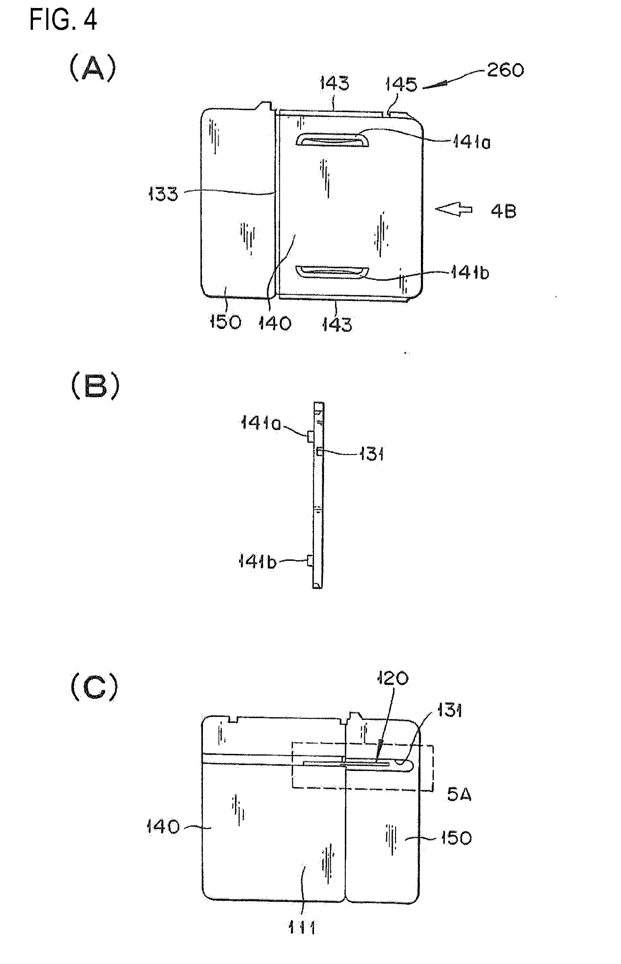

Referring to Figs. 3 and 4, the sensor member 110a has the sensor 120, the retaining portion 130 which retains the sensor 120 and the electronic circuit 160 which is accommodated in the retaining portion 130. The sensor 120 is accommodated in a groove-shaped concave guide 131 provided in the retaining portion 130. The concave guide 131 is formed in a back surface 111 of the sensor member 110 along a longitudinal direction. The back surface 111 of the sensor member 110 is a surface on a side on which the sensor 120 is arranged to face with the skin s of the living body when the sensor 120 is placed (see Fig. 17(B)).

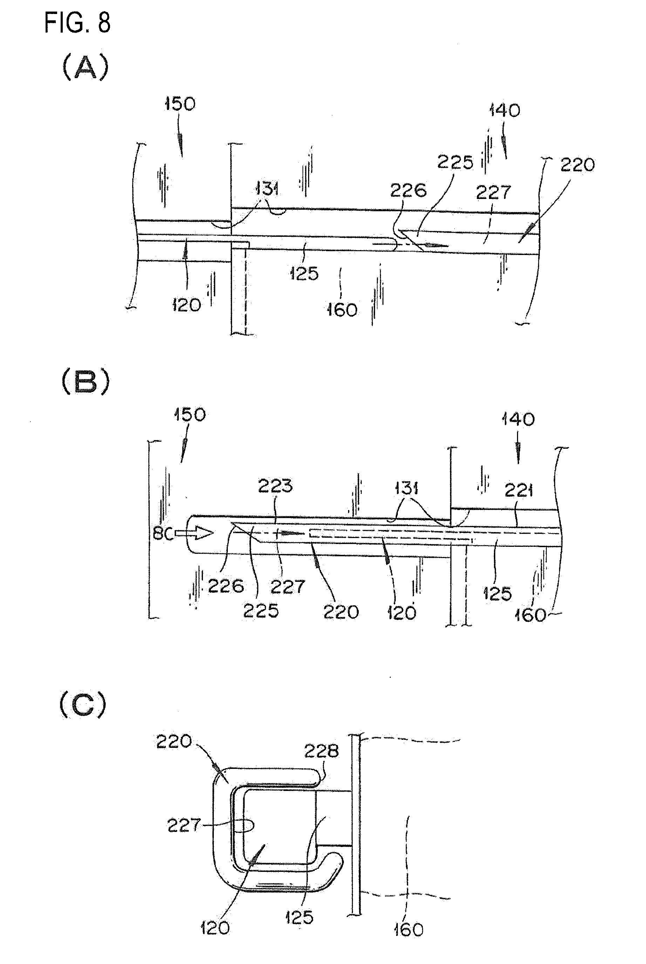

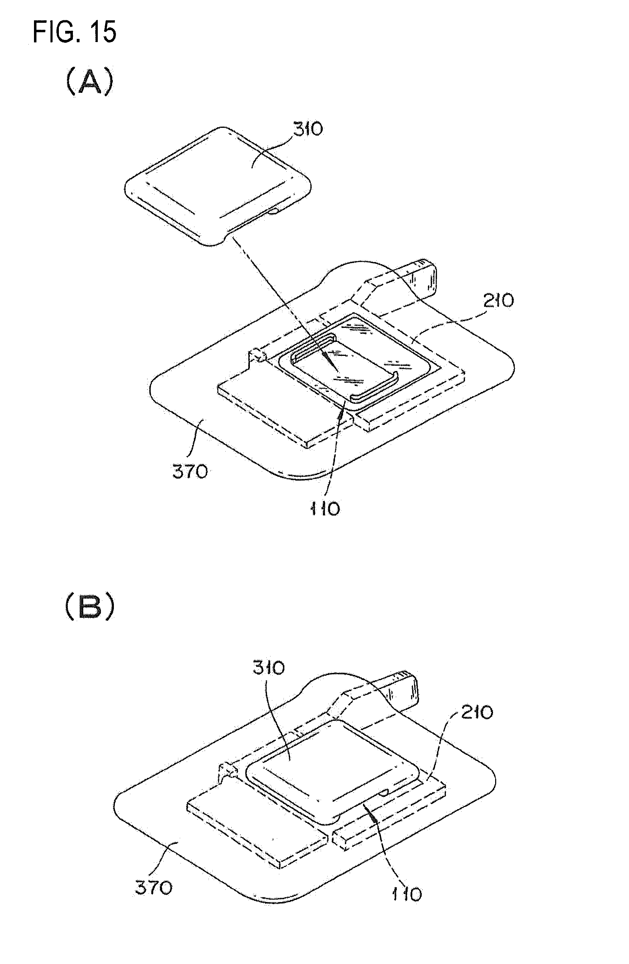

The sensor member 110 is prepared in a state in which the sensor member 110 is connected to the needle assembly 210 prior to installation of the sensor 120 to the living body (see Fig. 15(A)). When the needle assembly 210 is connected, the guide needle 220 which guides insertion of the sensor 120 and the sensor 120 are assembled (see Fig. 8(B)).

The retaining portion 130 of the sensor member 110 has the main body portion 140 which covers and retains a proximal portion 221 side of the guide needle 220, a movable portion 150 which covers and retains a needle portion 225 formed on a distal portion 223 side of the guide needle 220, and a hinge (corresponding to a jointing member) 133 which rotatably joints the main body portion 140 and the movable portion 150 about the axial direction which crosses the longitudinal direction of the guide needle 220. Part of the main body portion 140 of the retaining portion 130 is made thin and this site is formed to have a bendable structure to have the bendable site function as the hinge 133.

The retaining portion 130 can be manufactured using the same material as a material which forms the case 313 of the signal processing circuit member 310, and, for example, hard plastic, metal or ceramic can be adequately selected for the retaining portion 130.

When the sensor member 110 and the needle assembly 210 are connected, the guide needle 220 is accommodated in the concave guide 131 of the sensor member 110, and the sensor 120 is accommodated in the guide needle 220 (see Figs. 8(A) and 8(B)). Following connection of the needle assembly 210 and the sensor member 110, the proximal portion 221 side of the guide needle 220 is held by the main body portion 140 of the retaining portion 130. Meanwhile, the needle portion 225 formed on the distal portion 223 side of the guide needle 220 is retained by the movable portion 150 of the retaining portion 130.

By rotating the movable portion 150 through the hinge 133 in a state in which the guide needle 220 is retained by the retaining portion 130, it is possible to expose the needle portion 225 of the guide needle 220 (an arrow r in Figs. 3 (B) and 17(A) indicates rotation). Further, by rotating the movable portion 150 in a direction opposite to the rotation direction upon exposure in the state in which the needle portion 225 of the guide needle 220 is exposed, the needle portion 225 of the guide needle 220 can be covered and shrouded by the movable portion 150.

The main body portion 140 of the sensor member 110 has the fitting ribs 141a and 141b which connect with the signal processing circuit member 310, slide rails 143 which connect with the needle assembly 210 and a lock concave portion 145 which fixes the needle assembly 210. The electronic circuit 160 which transmits and receives a signal to and from the sensor 120 is accommodated in advance in the main body portion 140, and is prepared.

The slide rails 143 are provided to fit to a slide guide 250 of the needle assembly 210. By using the slide rails 143, it is possible to slide and connect the sensor member 110 with respect to the needle assembly 210 (an arrow a in Fig. 6(B) indicates sliding). The shape and the number of installation of slide rails 143 are not limited in particular, and can be adequately changed as long as the sensor member 110 and the needle assembly 210 can be smoothly attached and the sensor 120 can be smoothly accommodated in the guide needle 220.

The lock concave portion 145 is a site to which a lock protrusion 261 of the needle assembly 210 is fitted (see Figs. 4(A) and 6(A)). The lock concave portion 145 and the lock protrusion 261 form a lock member 260. By using the lock member 260, it is possible to mutually fix the needle assembly 210 and the sensor member 110. By this means, it is possible to prevent the needle assembly 210 from being unexpectedly separated from the sensor member 110.

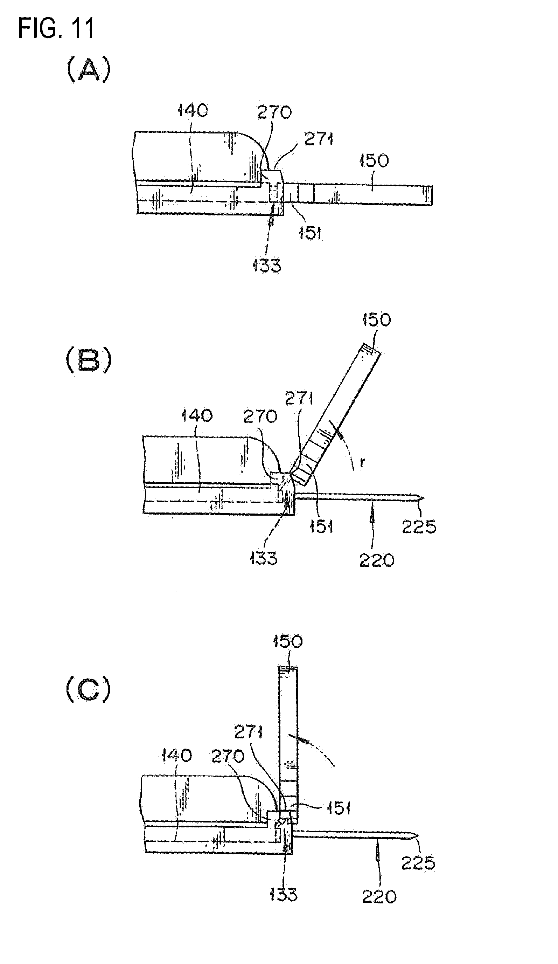

The movable portion 150 has a protrusion portion 151 which adjusts a rotation angle of the movable portion 150. The protrusion portion 151 is used in combination with an angle adjusting structure 270 provided in the needle assembly 210 (see Fig. 11). When the movable portion 150 is rotated on the hinge 133, it is possible to pull the movable portion 150 upright in a direction vertical to a planar direction of the main body portion 140 of the sensor member 110 and maintain this state.

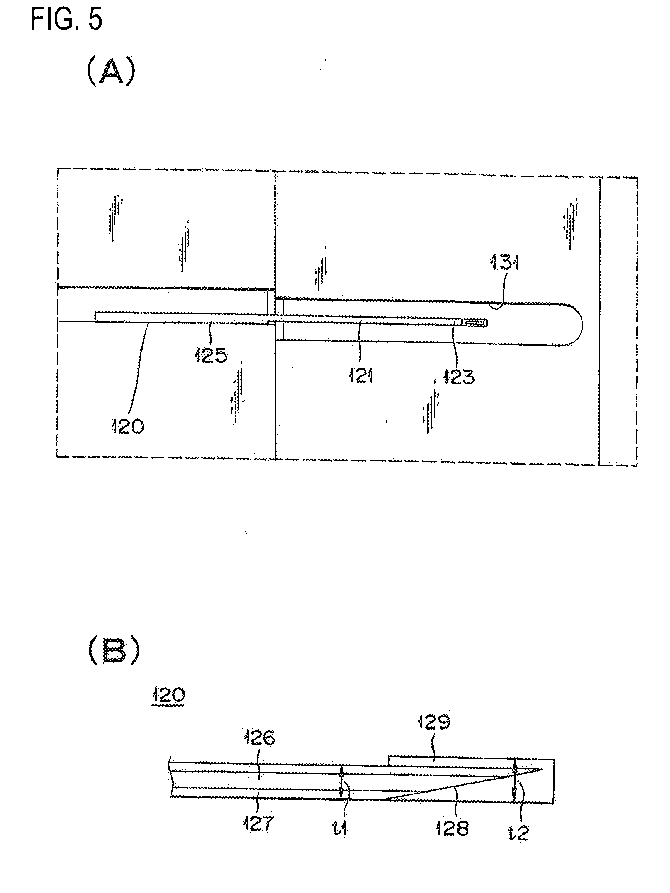

Referring to Fig. 5(A), the sensor 120 has an elongated outer shape which can be accommodated in the concave guide 131 of the sensor member 110. The sensor 120 has a flexible sensor lead portion 121, a sensor probe 123 provided at a tip of the sensor lead portion 121 and a circuit lead portion (corresponding to a connecting member) 125 which is electrically connected with the electronic circuit 160 on the sensor member 110. The sensor 120 is retained by the retaining portion 130 in a state in which the sensor 120 is electrically connected with the electronic circuit 160 accommodated in the main body portion 140 through the circuit lead portion 125 (see Fig. 8(C)).

When communication between the sensor member 110 and the signal processing circuit member 310 is performed without an electrical contact such as magnetism as described above, the sensor 120 can transmit a signal matching detected analyte components to the electronic circuit 160 accommodated in the sensor member 110 where necessary. This electronic circuit 160 has a microcomputer where necessary and, after predetermined processing in the microcomputer, transmits the signal to the signal processing circuit member 310 attached to the sensor member 110. The electronic circuit 311 of the signal processing circuit member 310 processes the received signal, and transmits a measurement result matching the signal to the user. A method of transmitting the measurement result to the user can adequately adopt a method of visually transmitting information by means of, for example, a display or a method of auditorily transmitting information by means of, for example, an alarm.

The sensor probe 123 produces a signal matching an existence of the analyte components of the living body or a concentration of the analyte components. The sensor probe 123 can adequately select a signal from various signals according to the analyte components. When, for example, a glucose concentration is measured using glucose oxidative enzyme (GOD), it is possible to use an oxidation-reduction electrode with the GOD fixed to the electrode. Further, it is possible to adopt a mode which uses a fluorescent pigment which emits fluorescence by being coupled to glucose or decreases fluorescence. In addition, it is also possible to employ a configuration of measuring a physical amount such as a temperature by means of, for example, an analysis semiconductor chip and producing a predetermined signal based on this measurement result.

Although a size of the sensor probe 123 can be adequately changed according to a measurement target analyte component and a unit which extracts a signal, when, for example, the sensor probe 123 has an outer shape of a cuboid, a cross-sectional dimension is preferably 3 mm x 3 mm or less. Particularly, the cross-sectional dimension is preferably 0.05 mm x 0.05 mm or more to 1 mm x 1 mm or less. The length is preferably, for example, about 0.1 mm to 20 mm.

When the sensor 120 is an electrode sensor, a conductor which can transmit an electrical signal can be arranged in the sensor 120. The conductor is laid to the electronic circuit 160 arranged in the sensor member 110 through the sensor lead portion 121 and the circuit lead portion 125 from the sensor probe 123. For the conductor, it is possible to use bundled copper wires of a relatively small diameter or use a flexible polyimide with a printed wiring.

When a sensor probe is adopted which can obtain as a signal a change in optical characteristics using a pigment which emits fluorescence by being coupled to analyte or a (for example, a pH sensor) pigment which absorbs light of a specific wavelength, it is possible to use a structure of an optical waveguide instead of the conductor. Fig. 5(B) illustrates the sensor 120 in which an optical waveguide core 126 made of glass, acrylic and cyclic polyolefin and the like, an optical waveguide clad 127 of fluorine resin having a lower refractive index than that of the optical waveguide core 126, a diffuse reflection surface 128 which is provided on a tip surface side of the optical waveguide core 126 and the optical waveguide clad 127 and which diffuses light produced from a circuit portion and light produced from a pigment and a pigment layer 129 which is arranged to face with this diffuse reflection surface 128 across the optical waveguide clad 127 and interacts with the analyte components are connected.

The diffuse reflection surface 128 is made by scraping the tip of the optical waveguide clad 127 to have a concavity or a convexity, and spattering, plating or adhering a diffuse reflecting member having a high optical reflectivity such as aluminum. A thickness t2 of a site corresponding to the sensor probe portion can be manufactured greater than a thickness t1 of a portion leading to the circuit lead portion from the sensor lead portion.

Referring to Figs. 6 to 8, the needle assembly 210 has the guide needle 220, a convex guide 230 which accommodates the proximal portion 221 side of the guide needle 220 and in which a notch 231 which exposes part of the guide needle 220 is formed, a concave surface 240 on which the sensor member 110 is placed, the slide guides 250 to which the slide rails 143 of the sensor member 110 can fit, a lock protrusion 261 which forms the lock member 260, the angle adjusting structure 270 which adjusts the angle of the movable portion 150, a lever 280 which releases fixation of the fixed needle assembly 210 and sensor member 110 and a flexible portion 281 which is provided to be flexibly deformed to smoothly release fixation.

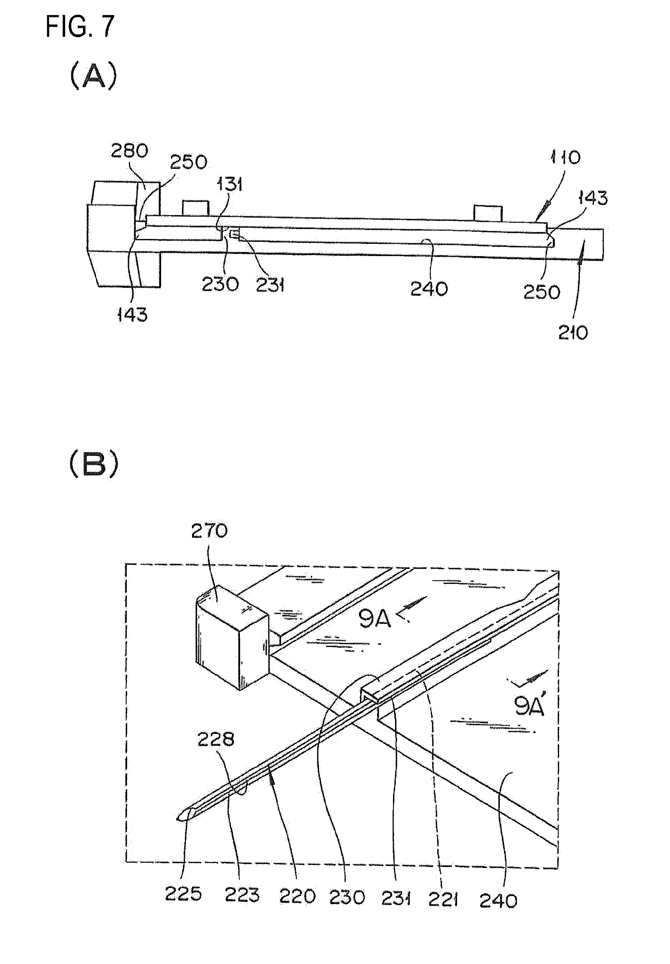

The guide needle 220 has the needle portion 225 which is formed in a sharp tip shape to smoothly perform a puncturing operation to insert the guide needle 220 in the living body, an opening end 226 which is formed in the distal portion 223, an internal space 227 which movably accommodates the sensor 120 and a slit 228 which is formed to extend from the opening end 226 along the longitudinal direction (see Figs. 7(B) and 8). In the guide needle 220, the slit 228 is formed in a general stainless puncturing needle for medical use.

The sensor member 110 and the needle assembly 210 are connected and separated to relatively slide the both members 110 and 210. By using the slide rails 143 provided on the sensor member 110 side and the slide guides 250 provided on the needle assembly 210 side, it is possible to easily and smoothly perform connecting and separating operations by way of sliding.

The sensor member 110 and the needle assembly 210 are connected by sliding the sensor member 110 with respect to the needle assembly 210 along an arrow a in Fig. 6(B). In this case, the slide rails 143 of the sensor member 110 are guided by the slide guides 250 of the needle assembly 210 and fitted. The sensor 120 is accommodated in the internal space 227 through the opening end 226 of the guide needle 220 in a state in which the sensor 120 is retained by the retaining portion 130 of the sensor member 110 (see Figs. 8(A) and 8(B)). Upon connection, the lock concave portion 145 provided in the main body portion 140 of the sensor member 110 and the lock protrusion 261 of the needle assembly 210 are fitted, so that the sensor member 110 and the needle assembly 210 are fixed.

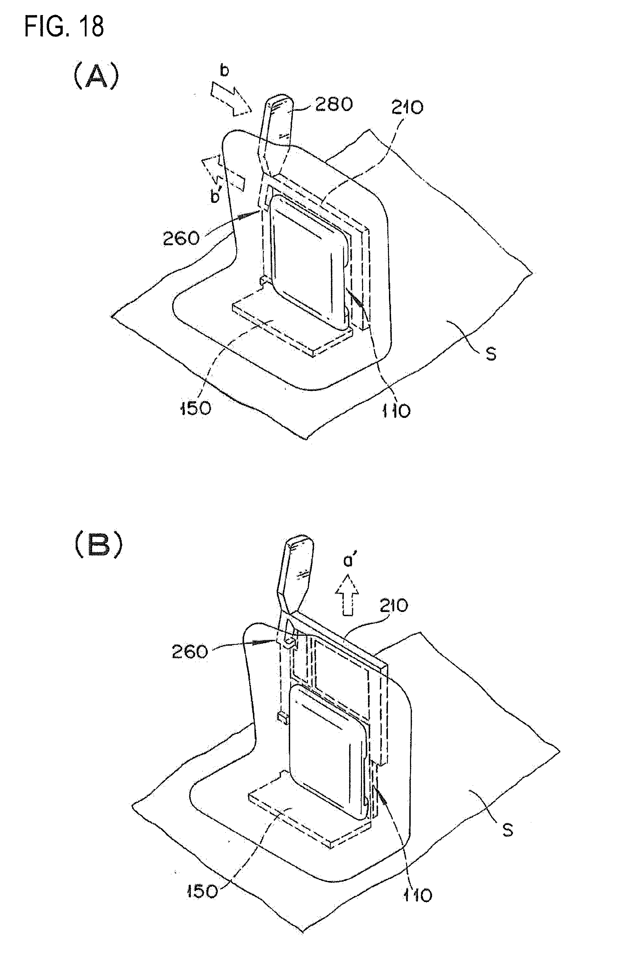

The sensor member 110 and the needle assembly 210 are separated by sliding the needle assembly 210 with respect to the sensor member 110 along an arrow a' in Fig. 6 (B) (see also Fig. 18(B)). Upon separation, the lever 280 is pushed in along an arrow b in Fig. 6 (B) (see also Fig. 18 (A)). The lock protrusion 261 provided in the needle assembly 210 is pulled out from the lock concave portion 145 provided in the sensor member 110 starting from the flexible portion 281 jointed to the lever 280, so that fixation of the sensor member 110 and the needle assembly 210 is released. The fixation is released, so that it is possible to slide the needle assembly 210 with respect to the sensor member 110. Following sliding, the sensor member 110 and the needle assembly 210 are separated, and the sensor 120 is pulled out from the internal space 227 of the guide needle 220. After the guide needle 220 which accommodates the sensor 120 punctures the living body and the needle assembly 210 is separated, only the sensor 120 is left beneath the skin of the living body.

The sensor member 110 and the needle assembly 210 are provided to relatively slide along the longitudinal direction of the guide needle 220, it is possible to prevent the guide needle 220 and the sensor 120 from being bent or caught upon connection and separation of the sensor member 110 and the needle assembly 210. It is possible to smoothly accommodate the sensor 120 in the guide needle 220 and remove the guide needle 220 from the living body.

When the sensor member 110 and the needle assembly 210 are slid, the circuit lead portion 125 of the sensor 120 is inserted in the slit 228 formed in the guide needle 220 (see Fig. 8(C)). While the circuit lead portion 125 is guided by the slit 228, the sensor member 110 and the needle assembly 210 are slid. It is possible to prevent the circuit lead portion 125 of the sensor 120 and the guide needle 220 from interfering each other upon sliding and, consequently, smoothly perform connecting and separating operations upon sliding.

The lever 280 can be adequately attached an attachment member 282 as indicated by a broken line in Fig. 6(A). By attaching the attachment member 282, it is possible to perform an operation of releasing fixation using one of user's hands or both of left and right hands. By this means, it is possible to further improve operability of the separating operation.

Referring to Fig. 7(B), the needle portion 225 formed at the distal of the guide needle 220 is arranged to be exposed from the convex guide 230 of the needle assembly 210. An exposure length at which the guide needle 220 is exposed from the convex guide 230 is preferably, for example, about 1 mm to 30 mm and, more preferably, about 5 mm to 10 mm. According to the exposure length set in this way, the guide needle 220 can be inserted in and beneath the skin, and the exposure length becomes a puncturing length of the guide needle 220 in the living body.

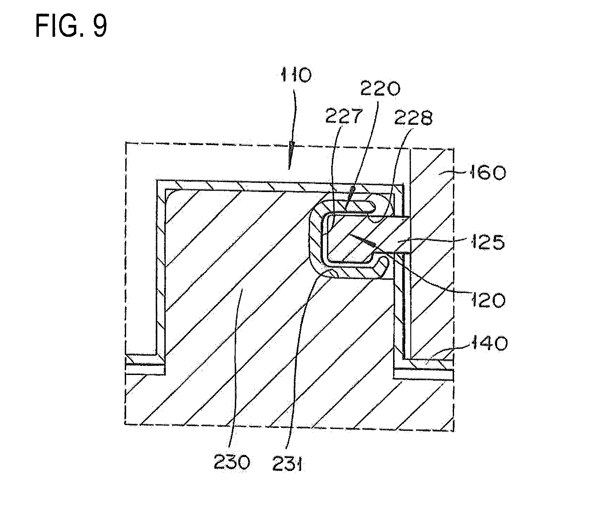

Fig. 9 is an enlarged cross-sectional view of a vicinity of the circuit lead portion 125 of the sensor 120. Fig. 9 shows a state in which the sensor 120 is accommodated in the guide needle 220 in which the slit 228 is formed. The circuit lead portion 125 of the sensor 120 is led from the slit 228 of the guide needle 220 and is arranged. Before and after the sensor member 110 and the needle assembly 210 are connected or separated, it is possible to keep electrical connection between the electronic circuit 160 accommodated in the main body portion 140 of the retaining portion 130 which forms the housing of the sensor member 110 and the circuit lead portion 125 of the sensor 120.

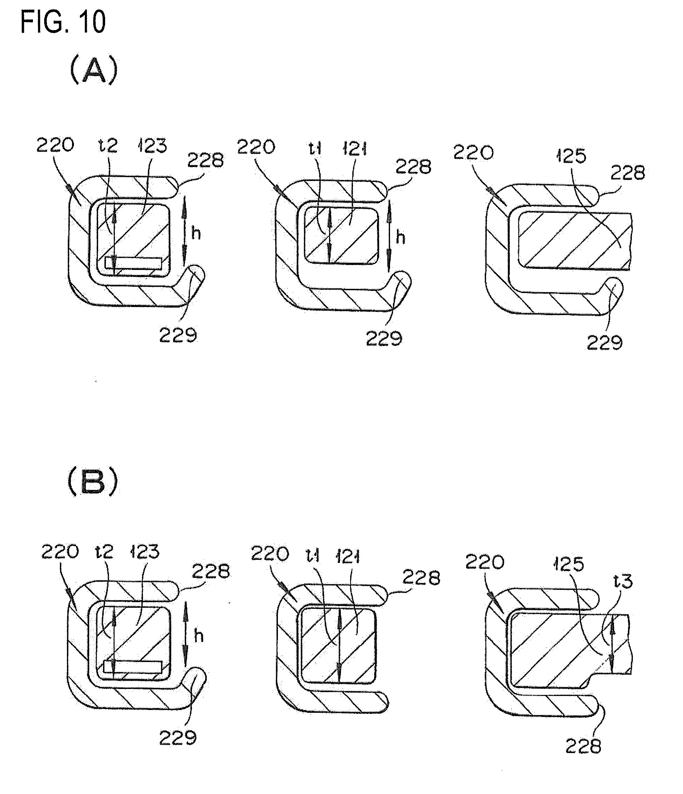

A cross-sectional shape of the guide needle will be described with reference to Fig. 10.

From the left, Figs. 10(A) and 10(B) both are cross-sectional views of a guide needle distal portion in which the sensor probe 123 is arranged, a center portion of the guide needle which connects the sensor probe and the circuit lead portion 125 and a guide needle proximal portion in which the circuit lead portion 125 is arranged.

Referring to Fig. 10(A), at a site at which the sensor probe 123 of the sensor 120 is arranged, a burr 229 is formed in the guide needle 220. The burr 229 is formed in the guide needle 220 and the thickness t2 of the sensor probe 123 is formed larger than the slit width h of the guide needle 220 (h < t2) to prevent the sensor 120 from slipping from the guide needle 220. The burr 229 of the guide needle 220 can also be partially formed at a site at which the sensor lead portion 121 of the sensor 120 is arranged or at a site at which the circuit leadportion 125 of the sensor 120 is arranged, or formed in the entire length in the longitudinal direction of the guide needle 220.

Although the slit width h of the guide needle 220 can be adequately changed according to, for example, an outer shape of the guide needle 220, the slit width h is preferably formed to have, for example, about 0.04 mm to 2.9 mm. When the slit 228 having the above slit width h is formed, the thickness t2 of the sensor probe 123 is preferably formed to, for example, about 0.05 mm to 3.0 mm. Further, when the sensor probe 123 having the above thickness t2 is formed, the thickness t1 of the sensor lead portion 121 is preferably formed to, for example, about 0.01 mm to 2.8 mm.

Referring to Fig. 10(B), a configuration can be employed where, for example, the burr 229 is formed only in a vicinity of a site of the guide needle 220 at which the sensor probe 123 is arranged, and a site (t3 < h) which is thinner than the slit width h of the guide needle 220 is formed in the circuit lead portion 125 of the sensor 120 is formed. By employing this configuration, it is possible to prevent the sensor probe 123 from slipping from the guide needle 220 and lead the circuit lead portion 125 outside the guide needle 220 and, consequently, it is not necessary to form the burr 229 extending along the entire guide needle 220. Further, by, for example, using in combination the sensor 120 having the size illustrated in Fig. 10(B) and the guide needle 220 having the shape illustrated in Fig. 10(A), it is possible to suitably prevent the sensor lead portion 121 of the sensor 120 from dropping from the slit 228.

Next, a function of the angle adjusting structure provided in the needle assembly will be described.

Referring to Fig. 11(A), the movable portion 150 is lifted on the hinge 133 in a state prior to rotation of the movable portion 150.

Referring to Fig. 11(B), the movable portion is further rotated on the hinge 133. The protrusion portion 151 of the movable portion 150 gets over a corner portion 271 of the angle adjusting structure 270 of the needle assembly 210. In this case, a side surface of the protrusion portion 151 and a side surface of the angle adjusting structure 270 are placed in point contact and the movable portion 150 is pulled toward the sensor member 110 side (a lower side in Fig. 11) by the hinge 133, so that a force to rotate the movable portion 150 in an opposite direction applies. In this state, it is difficult to keep a predetermined rotation angle of the movable portion 150.

Referring to Fig. 11(C), when the protrusion portion 151 of the movable portion 150 completely gets over the corner portion 271 of the angle adjusting structure 270, a side surface of the movable portion 150 is placed on the corner portion 271. It is possible to keep a state in which the movable portion 150 is lifted in a direction orthogonal to the planar direction of the main body portion 140 of the sensor member 110.

Next, a connection mode of the sensor member and the signal processing circuit member will be described.

Referring to Fig. 12, the sensor member 110 and the signal processing circuit member 310 are connected by fitting the fitting ribs 315a and 315b formed in the signal processing circuit member 310 to the fitting ribs 141a and 141b formed at positions at which the concave guide 131 of the sensor member 110 is sandwiched. In a state in which a planar direction of the sensor member 110 and a planar direction of the signal processing circuit member 310 are parallel (see Fig. 12(A)), both members 110 and 310 are hardly separated. That is, it is possible to suitably prevent the sensor member 110 and the signal processing circuit member 310 from being unexpectedly separated in a state in which the sensor system 100 is fixed to the skin s in the living body.

To separate the sensor member 110 and the signal processing circuit member 310, part of the vicinity of the fitting ribs 141a and 141b of the sensor member 110 is deformed by way of bending starting from the concave guide 131 of the sensor member 110 (see Fig. 12(B)). By this means, it is possible to easily remove the signal processing circuit member 310. The electronic circuit 160 which processesandtransmits a signal transmitted from the sensor 120 is built in the sensor member 110, and therefore it is difficult to significantly deform the entire sensor member 110. In the concave guide 131 formed in the main body portion 140 of the sensor member 110 and the movable portion 150 of the sensor member 110, the sensor 120 which can flexibly deform is arranged with a gap. Consequently, there is no concern that the function of the sensor system 100 is immediately impaired following deformation of a portion near the concave guide 131, and it is possible to separate the sensor member 110 and the signal processing circuit member 310 while deforming the concave guide 131 to extend along the longitudinal direction.

The shapes and the numbers of the fitting ribs 141a and 141b formed in the sensor member 110 and the fitting ribs 315a and 315b formed in the signal processing circuit member 310 are not limited in particular, and can be adequately changed. In the illustrated example, two fitting ribs 141a and 141b, and 315a and 315b are prepared as pairs and have different shapes such that a positional relationship between the sensor member 110 and the signal processing circuit member 310 upon connection is fixed at all times. Further, to prevent left and right positions in Fig. 12 from being connected by error, the fitting ribs 141a and 141b, and the fitting ribs 315a and 315b are formed to have different sizes. Furthermore, even in a state in which the sensor member 110 is fixed to the skin s, that is, in a state in which there is the skin s contacting the lower surface of the sensor member 110 (see Fig. 19(B)), it is possible to easily perform the separating operation without labor by pressing an end portion of the sensor member 110 downward as indicated by an arrow in Fig. 12(B) and peeling the signal processing circuit member 310 upward.

Referring to Figs. 13 and 14, the fixing member 350 which fixes the sensor member 110 to the skin s of the living body has a first adhesive portion 351 which is provided on a back surface of the main body portion 140 of the sensor member 110, a second adhesive portion 352 which is provided on a back surface of the movable portion 150 of the sensor member 110 and a sensor side peeling member 360 which covers the first adhesive portion 351 and the second adhesive portion 352 and is peelably attached (see Figs. 14(A) and 14(B)). The back surface of the main body portion 140 and the back surface of the movable portion 150 are surfaces on a side on which the sensor system 100 is arranged to face with the skin s of the living body when the sensor system 100 is placed, that is, surfaces positioned on the back surface 111 side of the sensor member 110 (see Fig. 17(B)).

The sensor side peeling member 360 has a first peeling piece 361 which is attached to the first adhesive portion 351 and a second peeling piece 362 which is separated from the first peeling piece 361 and is attached to the second adhesive portion 352. The first adhesive portion 351 and the second adhesive portion 352 are formed by pasting a base material which has an adhesive surface which can adhere to the living body and which is formed using a known adhesive, to the back surface 111 of the sensor member 110. The first peeling piece 361 and the second peeling piece 362 are formed by known peeling members in which peeling layers arranged to face with the adhesive surface of the base material are formed.

By detaching the first peeling piece 361, the first adhesive portion 351 is exposed. By placing the back surface of the main body portion 140 in contact with the skin s of the living body in a state in which the first adhesive portion 351 is exposed, the main body portion 140 is fixed to the living body (see Fig. 18(B)). Similarly, by detaching the second peeling piece 362 and placing the back surface of the movable portion 150 in contact with the skin s of the living body in a state in which the second adhesive portion 352 is exposed, the movable portion 150 is fixed to the living body (see Fig. 17(B)).

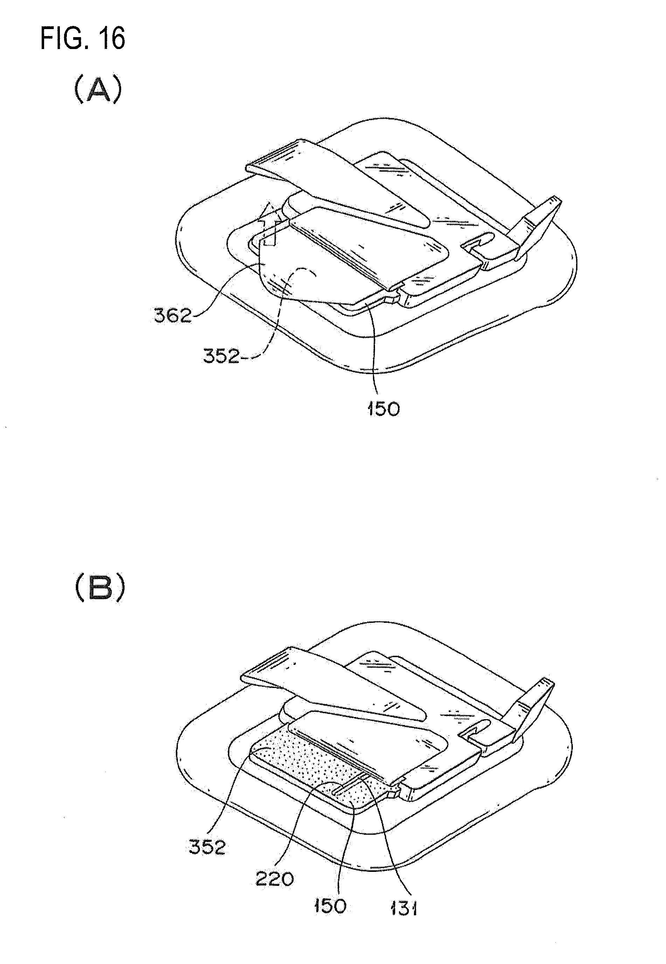

It is possible to independently peel the first peeling piece 361 and the second peeling piece 362. When the guide needle 220 punctures the skin s, the movable portion 150 is pressed against the skin s (see Fig. 16(B)), so that it is possible to fix the movable portion 150 to the skin upon the puncturing operation by peeling in advance the second peeling piece 362 prior to the puncturing operation. Further, the second peeling piece 362 attached to the back surface of the movable portion 150 is arranged to cover the concave guide 131 provided on the back surface of the movable portion 150, so that it is possible to prevent the needle portion 225 of the guide needle 220 from protruding from the concave guide 131 before the puncturing operation and improve safety upon use. Furthermore, it is possible to prevent the sensor 120 retained in the concave guide 131 from being contaminated.

The first peeling piece 361 has a sensor side tongue portion 363 which joints the first peeling piece 361 to the needle assembly 210. The sensor side tongue portion 363 is formed by extending part of the peeling member, and one end portion thereof is adhered to the needle assembly 210 (see Fig. 14(A)). When the needle assembly 210 is detached from the sensor member 110, the sensor side tongue portion 363 is pulled in a direction in which the needle assembly 210 moves in conjunction with sliding of the needle assembly 210 (an arrow a' in Fig. 14 indicates sliding). In conjunction with detachment of the needle assembly 210, the first peeling piece 361 is peeled from the first adhesive portion 351 through the sensor side tongue portion 363.

The fixing member 350 has a cover member 370 which has an outer periphery portion 371 arranged protruding from the sensor member 110 and covers an outer surface of the sensor member 110, a cover member side adhesive portion 372 which is provided on the surface of the outer periphery portion 371 which is arranged to face with the living body and a cover member side peeling member 373 which covers the cover member side adhesive portion 372 and is peelably attached (see Fig. 14(C)).

The cover member 370 is prepared in a state in which the cover member 370 is adhered to the surface of the sensor member 110 to prevent the cover member 370 from detaching from the sensor member 110. For the cover member 370, a soft resin film such as polyester, polyurethane, polyethylene or nylon can be used. The cover member side adhesive portion 372 is formed using the same material as those of the above-described first adhesive portion 351 and the second adhesive portion 352. The cover member side peeling member 373 is formed using the same material as that of the above-described sensor side peeling member 360.

The cover member side peeling member 373 has a cover member side tongue portion 374 which joints the cover member side peeling member 373 to the needle assembly 210 (see Fig. 14(C)). The cover member side tongue portion 374 is formed by extending part of the peeling member, and one end portion thereof is attached to the needle assembly 210. When the needle assembly 210 is detached from the sensor member 110, the cover member side tongue portion 374 is pulled in a direction in which the needle assembly 210 moves, in conjunction with movement of the needle assembly 210 (an arrow a' in Fig. 14 indicates sliding). In conjunction with detachment of the needle assembly 210, the cover member side peelingmember 373 is peeled from the cover member side adhesive portion 372 through the cover member side tongue portion 374.

In this manner, the sensor system 100 has the first adhesive portion 351 provided in the main body portion 140, the second adhesive portion 352 provided in the movable portion 150 and the cover member side adhesive portion 372 provided in the cover member 370 function as the fixing member 350. The sensor system 100 is, for example, distributed as a product to which the first peeling piece 361, the second peeling piece 362 and the cover member side peeling member 373 are attached in advance.

Next, a function of the sensor system according to the embodiment will be described.

Referring to Figs. 15(A) and 15(B), the sensor system 100 to which the sensor member 110 and the needle assembly 210 are attached is prepared. Prior to the puncturing operation of the guide needle 220, the signal processing circuit member 310 is attached to the sensor member 110. The sensor member 110, the needle assembly 210 and the signal processing circuit member 310 are integrated.

Referring to Figs. 16 (A) and 16(B), the second peeling piece 362 which is provided on the back surface of the movable portion 150 and which covers the second adhesive portion 352 is detached (indicated by an arrow in Fig. 16). The needle portion 225 of the guide needle 220 is shrouded and kept until the movable portion 150 is rotated, so that it is possible to safely handle the sensor system 100.

Referring to Fig. 17(A), the movable portion 150 of the sensor member 110 is rotated using the hinge 133 (an arrow r in Fig. 17 indicates rotation). Following rotation, the needle portion 225 of the guide needle 220 is exposed. It is possible to prepare for the puncturing operation according to a simple operation of rotating the movable portion 150.

When the movable portion 150 is rotated, the protrusion portion 151 provided in the movable portion 150 is arranged on the corner portion 271 of the angle adjusting structure 270 (see Fig. 11(C)). By utilizing the angle adjusting structure 270, it is possible to pull the movable portion 150 upright in a state vertical to the planar direction of the main body portion 140 of the sensor member 110.

Referring to Fig. 17(B), the user punctures the skin s by means of the guide needle 220 while pinching the sensor system 100 by the fingers of one hand. It is possible to perform the puncturing operation according to a simple operation of pressing the movable portion 150 against the skin s while visually checking the needle portion 225 of the guide needle 220 and a puncturing position. By this means, it is possible to place the sensor 120 at a target site. At the same time as the puncturing operation, the movable portion 150 is fixed to the skin s through the second adhesive portion 352.

Referring to Fig. 17(C), when the user pushes the lever 280 by the finger of one hand, fixation of the needle assembly 210 and the sensor member 110 fixed by the lock member 260 is released.

Referring to Fig. 18(A), the lock member 260 is released by pushing the lever 280 down in an arrow b direction and lifting the lock protrusion 261 in an arrow b' direction through the flexible portion 281 continuing to the lever 280. The fixation of the needle assembly 210 and the sensor member 110 is released, so that it is possible to slide the needle assembly 210 with respect to the sensor member 110.

Referring to Fig. 18(B), the user pinches the needle assembly 210 by the fingers of one hand and slides the needle assembly 210 (an arrow a' in Fig. 18 indicates sliding). The needle assembly 210 is slid to pull out the guide needle 220 from the skin s. Sliding is performed along the longitudinal direction of the guide needle 220, so that there is no concern that the guide needle 220 is bent and caught, and the guide needle 220 can be smoothly removed. Further, the circuit lead portion 125 of the sensor 120 is guided by the slit 228 formed in the guide needle 220, so that it is possible to keep electrical connection between the sensor 120 and the electronic circuit 160 before and after sliding (see Fig. 8(C)).

The sensor 120 is pulled out from the internal space 227 of the guide needle 220, and is left beneath the skin of the living body. By leaving the sensor 120 beneath the skin, it is possible to successively measure the analyte components in the living body.

The first peeling piece 361 which covers the first adhesive portion 351 provided on the back surface side of the main body portion 140 of the sensor member 110 is peeled off in conjunction with detachment of the needle assembly 210 from the sensor member 110. Similarly, the cover member side peeling member 373 which covers the cover member side adhesive portion 372 provided in the cover member 370 is peeled off in conjunction with detachment of the needle assembly 210 from the sensor member 110. It is possible to perform an operation of detaching the first peeling piece 361 and the cover member side peeling member 373 in conjunction with an operation of detaching the needle assembly 210 and, consequently, it is possible to reduce the number of operations required to place the sensor 120 and place the sensor 120 according to more simple process. Further, by peeling in advance the first peeling piece 361 and the cover member side peeling member 373 before fixing the sensor member 110 to the skin s, it is possible to fix the entire sensor member 110 to the skin s according to a simple operation of placing each of the first adhesive portion 351 and the cover member side adhesive portion 372 in contact with the skin s after detaching the needle assembly 210.

Referring to Fig. 19(A), after the needle assembly 210 is detached, the main body portion 140 of the sensor member 110 is rotated using the hinge 133 (an arrow r' in Fig. 19 indicates rotation). It is possible to fix the sensor member 110 according to the simple operation of placing the first adhesive portion 351 provided in the main body portion 140 of the sensor member 110 in contact with the skin s. In this case, although the sensor member 110 is bent following rotation of the main body portion 140, it is possible to use the sensor system 100 without being affected by bending, by setting a bent portion at a predetermined position or using a soft material which allows a bend.

When the main body portion 140 of the sensor member 110 is placed in contact with the skin s, the cover member side adhesive portion 372 provided in the outer periphery portion 371 of the cover member 370 is placed in contact with the skin s. The cover member 370 is fixed to the skin s through the cover member side adhesive portion 372. The sensor member 110 is fixed by the first and second adhesive portion 351 and 352 provided in the sensor member 110 and the sensor member 110 is fixed by the cover member side adhesive portion 372 provided in the cover member 370, so that it is possible to fix the sensor member 110 fast to the skin s. After fixation, the site of the cover member 370 adhered to the skin s functions as a sealing portion, and the vicinity of the sensor 120 embedded beneath the skin of the living body and an outer portion of the cover member 370 are insulated liquid tight. It is possible to prevent the sensor member 110 and the sensor 120 from being soaked wet and suitably prevent an error operation of the sensor 120 caused by infiltration of water. By this means, it is possible to successively measure analyte components in a state where the sensor 120 is placed in the living body.

Prior to installation of the sensor system 100, the signal processing circuit member 310 is attached to the sensor member 110, so that it is not necessary to separately attach the signal processing circuit member 310 after the installation. Placing the sensor 120 at a body surface portion which is hardly visually checked involves a complicated operation of connecting the signal processing circuit member 310 and there is a concern that a mistake of connection between the sensor 120 and the signal processing circuit member 310 is caused, the sensor system 100 allows the sensor 120 to be more easily placed in the living body without causing this problem.

The signal processing circuit member 310 which is detachably provided to the sensor member 110 can be commoditized with the different sensor member 110. Meanwhile, the sensor member 110 which is directly fixed to the skin s and the needle assembly 210 which includes the guide needle 220 which punctures beneath the skin can be disposed every time the sensor member 110 and the needle assembly 210 are used. The signal processing circuit member 310 which includes the electronic circuit 311 as a component is comparatively expensive, and the signal processing circuit member can be shared a plurality of times without disposing the signal processing circuit member, so that it is possible to reduce cost of the users.

As described above, the sensor system 100 according the present embodiment can perform a series of operations of placing the sensor 120 in the living body in a state in which the signal processing circuit member 310 which has the electronic circuit 311 which processes a signal is attached to the sensor member 110 which has the sensor 120, and, consequently, does not need to attach the signal processing circuit member 310 after the puncturing operation of the guide needle220. Consequently, evenwhentheuserplacesthesensor 120 at a body surface portion which the user can hardly visually check, it is possible to improve operability of the operation of placing the sensor 120 without causing complication of the operation of attaching the signal processing circuit member 310. By this means, it is possible to improve user-friendliness of the users who use the sensor system 100.

Further, the movable portion 150 covers and retains the needle portion 225 of the guide needle 220, so that it is possible to safely handle the sensor system 100 before the puncturing operation and prepare for the puncturing operation according to the simple operation of rotating the movable portion 150. Furthermore, it is possible to perform the puncturing operation of the guide needle 220 according to the simple operation of pressing the movable portion 150 against the skin s while visually checking the needle portion 225 of the guide needle 220 exposed from the movable portion 150 and the puncturing position.

Still further, it is possible to fix the sensor member 110 to the skin s of the living body according to the simple operation of only placing the first adhesive portion 351 provided on the back surface side of the main body portion 140 of the sensor member 110 and the second adhesive portion 352 provided on the back surface side of the movable portion 150 of the sensor member 110 in contact with the skin s.

Moreover, by peeling in advance the second peeling piece 362 which covers the second adhesive portion 352 provided on the back surface side of the movable portion 150 of the sensor member 110 prior to the puncturing operation, it is possible to fix the movable portion 150 to the skin s upon the puncturing operation. Further, by arranging the second peeling piece 362 to cover the guide needle 220 retained by the movable portion 150, it is possible to prevent the needle portion 225 of the guide needle 220 from protruding before the puncturing operation, and further improve safety upon use.

Furthermore, the first peeling piece 361 is peeled from the first adhesive portion 351 through the sensor side tongue portion 363 in conjunction with detachment of the needle assembly 210, so that it is possible to reduce the number of operations required to place the sensor 120, and place the sensor 120 according to more simple process. Still further, the first peeling piece 361 is peeled in advance before an operation of fixing the sensor member 110 to the skin s is performed, so that it is possible to fix the sensor member 110 according to a simple operation of rotating the main body portion 140 and placing the first adhesive portion 351 in contact with the skin s after detaching the needle assembly 210.

Moreover, the sensor member 110 and the needle assembly 210 are provided to connect and separate following relative sliding along the longitudinal direction of the guide needle 220, so that it is possible to prevent the guide needle 220 and the sensor 120 from being bent or caught upon connection or separation of the sensor member 110 and the needle assembly 210, and smoothly accommodate the sensor 120 in the guide needle 220 and remove the guide needle 220 from the living body.

Further, when the sensor member 110 and the needle assembly 210 are relatively slid, the circuit lead portion 125 of the sensor 120 can be guided by the slit 228 formed in the guide needle 220, so that it is possible to prevent the circuit lead portion 125 of the sensor 120 and the guide needle 220 from interfering each other. By this means, it is possible to smoothly perform connecting and separating operations by way of sliding.

Further, the sensor member 110 is fixed by the cover member side adhesive portion 372 provided in the cover member 370, so that it is possible to fix the sensor member 110 fast to the skin s of the living body. After the fixation, a site of the cover member 370 adhered to the skin s functions as the sealing portion, so that it is possible to prevent the sensor member 110 and the sensor 120 from being soaked wet, and suitably prevent an error operation of the sensor 120 caused by infiltration of water.

Further, the cover member side peeling member 373 is peeled from the cover member side adhesive portion 372 through the cover member side tongue portion 374 in conjunction with detachment of the needle assembly 210, so that it is possible to reduce the number of operations required to place the sensor 120 and place the sensor 120 according to more simple process. Furthermore, the cover member side peeling member 3 73 is peeled in advance before the sensor member 110 is fixed to the skin s, so that it is possible to fix the sensor member 110 according to a simple operation of placing the cover member side peeling member 373 in contact with the skin s after the needle assembly 210 is detached.

### <Modified Example of sensor and guide needle>

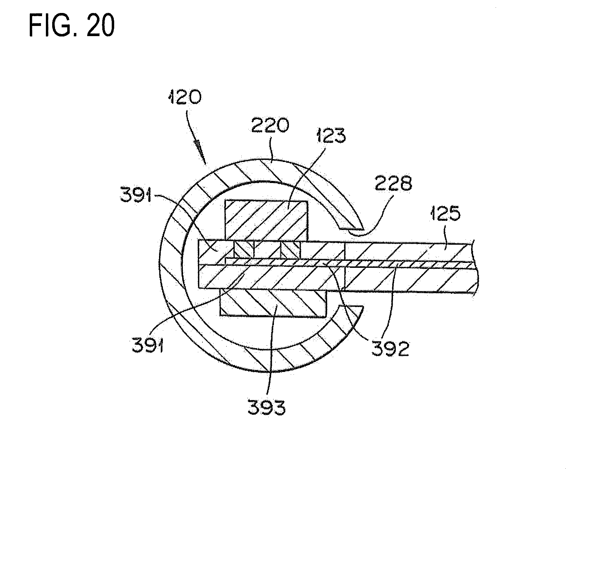

Referring to Fig. 20, in Modified Example, a cross-sectional shape of a guide needle 220 is formed in a generally C shape. Further, a sensor probe 123 of a sensor 120 converts a signal based on analyte components of a living body into an electrical signal. A conductive layer 392 which transmits the electrical signal to an electronic circuit 311 of a signal processing circuit member 310 side is sandwiched liquid tight by two flexible substrates 391.

A retaining layer 393 which adjusts flexibility of the flexible substrates 391 is provided and the sensor probe 123 of the sensor 120 has a predetermined thickness to prevent the sensor probe 123 from slipping from a slit 228 of the guide needle 220.

For a material of the retaining layer 393, for example, the flexible material such as polyimide, silicone rubber, nylon, fluorine resin or metal such as stainless steel as that of the flexible substrates 391 can be used.

The retaining layer 393 only needs to be placed close to the sensor probe 123 at least a predetermined position in the longitudinal direction of the sensor 120. Meanwhile, by providing the retaining layer 393 in the entire length of the sensor 120 along the longitudinal direction of the sensor 120, it is possible to provide adequate elasticity (resilience) to the sensor 120. By providing elasticity, it is possible to prevent the guide needle 220 from being caught by the sensor 120 upon detachment of a needle assembly 210 from a sensor member 110. Consequently, it is preferable to adopt a mode in which the retaining layer 393 is placed over the entire length of the sensor 120.

The above embodiment can be adequately changed.

For example, it is possible to employ a configuration of directly transmitting and receiving a signal between the sensor 120 and the electronic circuit 311 of the signal processing circuit member 310 side without an electronic circuit 160 on the sensor member 110 side by adding a signal processing function of the electronic circuit 160 of the sensor member 110 side to the electronic circuit 311 of the signal processing circuit member 310 side. It is not necessary to place the electronic circuit 160, and, consequently, it is possible to make the sensor member small or simple.

A fixing member can be adequately changed as long as the fixing member can fix the sensor member on a surface of a skin. For example, it is possible to employ a configuration of performing fixation using one of an adhesive portion provided to the sensor member and an adhesive portion provided to a cover member. Meanwhile, by adopting a fixing method using a first adhesive portion 351 provided to a main body portion 140 and a second adhesive portion 352 provided to a movable portion 150, it is possible to smoothly perform a fixing operation and it is more preferable to employ a configuration including the first adhesive portion 351 and the second adhesive portion 352. Further, it is also possible to employ, for example, a configuration to which a member which supports fixation such as belt which has a planar fastener is adequately added.

An outer shape, the cross-sectional shape and a material of the guide needle are not limited only to the mode described in the embodiment in particular, and can be adequately changed as long as the guide needle can guide insertion of the sensor to the skin of a living body. In addition to the guide needles which have generally U-shaped or generally C-shaped cross-sectional shapes described in the embodiment, guide needles having arc or polygonal cross-sectional shapes can be used. Further, a method of guiding the sensor by means of the guide needle is not limited only to the mode in particular of accommodating the sensor in the needle and guiding the sensor, it is possible to adopt, for example, a mode of assembling the sensor outside the guide needle and inserting the sensor in the living body following puncturing of the guide needle.

This application claims priority to Japanese Patent Application No. 2010-256105 filed on November 16, 2010, the entire contents of which are incorporated by reference herein. Reference Signs List

- 100: SENSOR SYSTEM,
- 110: SENSOR MEMBER,
- 111: BACK SURFACE OF SENSOR MEMBER,
- 120: SENSOR,
- 121: SENSOR LEAD PORTION,
- 123: SENSOR PROBE,
- 125: CIRCUIT LEAD PORTION (CONNECTING MEMBER),
- 130: RETAINING PORTION,
- 131: CONCAVE GUIDE,
- 133: HINGE (JOINTING MEMBER),
- 140: MAIN BODY PORTION,
- 150: MOVABLE PORTION,
- 210: NEEDLE ASSEMBLY,
- 220: GUIDE NEEDLE,
- 221: PROXIMAL PORTION OF GUIDE NEEDLE,
- 223: DISTAL PORTION OF GUIDE NEEDLE,
- 225: NEEDLE PORTION,
- 226: OPENING END,
- 227: INTERNAL SPACE,
- 228: SLIT,
- 260: LOCK MEMBER,
- 310: SIGNAL PROCESSING CIRCUIT MEMBER,
- 311: ELECTRONIC CIRCUIT,
- 350: FIXING MEMBER,
- 351: FIRST ADHESIVE PORTION,
- 352: SECOND ADHESIVE PORTION,
- 360: SENSOR SIDE PEELING MEMBER,
- 361: FIRST PEELING PIECE,
- 362: SECOND PEELING PIECE,
- 363: SENSOR SIDE TONGUE PORTION,
- 370: COVER MEMBER,
- 371: OUTER PERIPHERY PORTION,
- 372: COVER MEMBER SIDE ADHESIVE PORTION,
- 373: COVER MEMBER SIDE PEELING MEMBER,
- 374: COVER MEMBER SIDE TONGUE PORTION,
- s: SKIN OF LIVING BODY.

## Claims

1. A sensor system comprising:
a sensor member which includes a sensor which detects an analyte component of a living body and transmits a signal, and a retaining portion which retains the sensor;
a needle assembly which is detachably attached to the sensor member and which includes a guide needle which guides insertion of the sensor to the living body;
a signal processing circuit member which is detachably attached to the sensor member, and which includes an electronic circuit which processes the signal from the sensor; and
a fixing member which fixes the sensor member to a skin of the living body,
wherein the sensor is placed in the living body in a state in which the signal processing circuit member is attached to the sensor member.

2. The sensor system according to claim 1, wherein
the retaining portion includes:
a main body portion which covers and retains a proximal portion side of the guide needle;
a movable portion which covers and retains a needle portion formed on a distal portion side of the guide needle; and
a jointing member which rotatably joints the main body portion and the movable portion about an axial direction which crosses a longitudinal direction of the guide needle, and
exposure and coverage of the needle portion is switched by rotating the movable portion.

3. The sensor system according to claim 2, wherein
the fixing member includes:
a first adhesive portion which is provided on a surface of surfaces of the main body portion which is arranged to face with the living body;
a second adhesive portion which is provided on a surface of surfaces of the movable portion which is arranged to face with the living body; and
a sensor side peeling member which covers the first adhesive portion and the second adhesive portion and is peelably attached.

4. The sensor system according to claim 3, wherein
the sensor side peeling member includes a first peeling piece which is attached to the first adhesive portion and a second peeling piece which is separated from the first peeling piece and is attached to the second adhesive portion, and
the second peeling piece is peeled in advance prior to the insertion of the sensor to the living body.

5. The sensor system according to claim 4, wherein
the first peeling piece includes a sensor side tongue portion which joints the first peeling piece to the needle assembly, and
the first peeling piece is peeled through the sensor side tongue portion in conjunction with detachment of the needle assembly from the sensor member.

6. The sensor system according to any one of claims 1 to 5, wherein
the sensor member and the needle assembly can be connected and separated following relative sliding of the guide needle along a longitudinal direction,
the guide needle includes an opening end formed at the distal portion and an internal space which movably accommodates the sensor,
when the sensor member is relatively slid with respect to the needle assembly, the sensor member and the needle assembly are connected, and the sensor is accommodated in the internal space through the opening end, and
when the needle assembly is relatively slid with respect to the sensor member in a state in which the sensor member and the needle assembly are connected, the sensor member and the needle assembly are separated, and the sensor is pulled out of the internal space.

7. The sensor system according to claim 6, wherein
the sensor is retained by the retaining portion through a connecting member, and
the guide needle includes a slit which is formed to extend from the opening end along the longitudinal direction of the guide needle, and which guides the connecting member when the sensor member and the needle assembly relatively slide.

8. The sensor system according to any one of claims 1 to 7, wherein
the fixing member includes:
a cover member which includes an outer periphery portion arranged protruding from the sensor member and covers an outer surface of the sensor member;
a cover member side adhesive portion which is provided in a surface of the outer periphery portion arranged to face with the living body; and
a cover member side peeling member which covers the cover member side adhesive portion and is peelably attached.

9. The sensor system according to claim 8, wherein
the cover member side peeling member includes a cover member side tongue portion which joints the cover member side peeling member to the needle assembly, and
the cover member side peeling member is peeled off through the cover member side tongue portion in conjunction with the detachment of the needle assembly from the sensor member.

10. A method of using the sensor system according to claim 3, comprising:
rotating the movable portion in a state in which the needle portion of the guide needle is covered by the movable portion, and exposing the needle portion of the guide needle;
fixing the rotated movable portion by means of the second adhesive portion;
guiding the insertion of the sensor by means of the guide needle and then detaching the needle assembly from the sensor member;
rotating the main body portion toward the living body; and
fixingthemainbodyportionbymeans of the first adhesive portion.
